# EUROPEAN PATENT SPECIFICATION

(11) **EP 2 313 760 B1**
(45) Date of publication and mention of the grant of the patent: **16.05.2018**
(21) Application number: 08808211.0
(22) Date of filing: 30.07.2008
(51) Int. Cl.: G01N 21/31, A61B 5/00, A61B 5/145, A61B 5/1455

(54) **DIODE LASER DEVICE FOR THE NON-INVASIVE MEASUREMENT OF GLYCAEMIA**
DIODENLASERVORRICHTUNG ZUR NICHTINVASIVEN MESSUNG VON GLYKÄMIE
DISPOSITIF À LASER À DIODE POUR LA MESURE NON INVASIVE DE LA GLYCÉMIE

(43) Date of publication of application: 27.04.2011
(73) Proprietor: Zambaiti Parati S.P.A., 24021 Albino (BG) (IT)
(72) Inventor: TROMBETTA, Pietro, 22070 Montano Lucino (CO) (IT); LONDONI, Vittorio, 24122 Bergamo (IT)
(86) International application number: PCT/IT2008/000516
(87) International publication number: WO 2010/013264

(56) References cited:
- EP-A1- 1 576 921
- WO-A-2008/111102
- WO-A1-2009/107156
- DE-U1- 20 320 092
- JP-A- 2004 166 775
- US-A- 6 113 541
- US-A- 6 151 516

## Description

### FIELD OF THE INVENTION

The present invention is directed to a device for non-invasively determining a glycaemia value without pricking the skin of a finger of a patient and without taking a blood sample.

### BACKGROUND OF THE INVENTION

US6151516 A discloses non-invasive systems for monitoring blood glucose and other difficult to detect blood constituent concentrations, such as therapeutic drugs, drugs of abuse, carboxyhemoglobin, Methemoglobin, cholesterol, in a finger.

### SUMMARY OF THE INVENTION

A device as defined in appended claim 1. Preferred embodiments of the device are defined in the dependent claims.

### BRIEF DESCRIPTION OF THE DRAWINGS

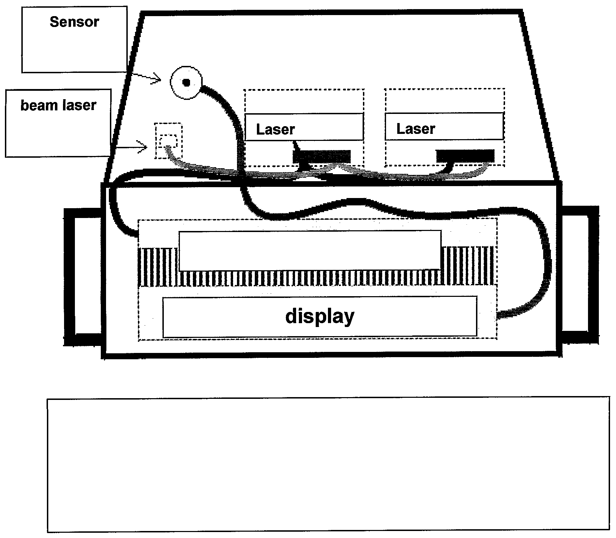

The FIGURE depicts a preferred embodiment of a device in accordance with the present invention.

### DETAILED DESCRIPTION OF PREFERRED EMBODIMENTS OF THE INVENTION

The device is built using two diode laser sources, with a conventional or with a drugged fibre source, fed either through the electric system in case the device is in its fixed form or by (rechargeable) batteries for the portable, Docket-size device.

The two diode laser sources involved are in a range between 500 and 1000 nm, with a power in the range between 0,01 to 100 mW.

The rays originating from the two diode lasers are conveyed through an optical condenser and, starting up a key, they are emitted either on a nail or on the skin or even on a free blood sample. A sensor comprising photodiodes or a CPU read the value of the radiation energy that is subtracted by the glycate haemoglobin and free plasmic glucose present in the blood. This value is converted into the immediate glycaemia value and appears on a device display.

The device can store in its memory up to 5000 of these values.

The device is provided with a cable interface, IR and Bluetooth® to connect and transmit the data to a PC.

The device allows to determine the glycaemia value without pricking the skin and without taking a blood sample.

More in detail, in the use and working of the present device, the patient simply has to put a finger into the device, operating a lever that opens a four-partitioned diaphragm.

Then, upon releasing the lever, a spring lets the diaphragm close, thus determining a centred arrangement of the finger, no matter its dimensions, on the focus of the two laser rays directed towards the photodiodes. (The laser device meets the requirements of Class safety features)

The device uses two diode laser sources between 500 and 1000 nm.

The patient switches the device on and the passing of the finger sets off an optical barrier that controls the diode laser starting.

Then the photodiodes read the values of the laser rays resulting from the selective absorption of the two laser rays, radiation energy by the glycate haemoglobin and free plasmic glucose present in the blood of the finger.

Moreover an analogic stage forwards these values to the processor that, after the stabilization of the system, digitises and normalises them, extracts a high number of them, strikes an average, determines the connections between them and, through a particular algorithm obtains the immediate glycaemia value. The device only needs a few seconds to determine the instantaneous glycaemia.

This being an absolutely painless and non-invading method, it can be used to get several glycaemia measurements, even in a very short time range, to monitor the trend through time, to check the effectiveness of a therapy, to build glycaemic curves of newborns, children, adults and elderly people.

The values are stored in sequence (up to 5000 assessments) and the memory can be displayed through two slide keys.

The device has a USB gate, a PC connection cable and is provided with a software to visualize and process the Therefore the device is easily activated by pressing the switch-on button.

In fact it is enough to put a finger into the device opening and start the centring system to obtain automatically, after a few seconds, the glycaemia value, that will be displayed on the device screen together with date and time of each measurement and the progressive registration number.

Moreover, the selective absorption of the two diode laser rays by the plasmic glucose and the glycate haemoglobin, that works as a control parameter, allows to obtain absolutely accurate glycaemia values. The two values are calculated in connection with each other, therefore the measurements is absolutely accurate notwithstanding the thickness and the colour of the skin and the thickness of the finger itself.

## Claims

1. A device for non-invasively determining a glycaemia value without pricking the skin of a finger of a patient and without taking a blood sample, comprising:
- two diode laser sources for emitting respective laser rays, said two diode laser sources being operable in a frequency range between 500 and 1000 nm, with a power in the range between 0,01 to 100 mW;
- a sensor comprising photodiodes for receiving the laser rays emitted by the two diode lasers and directed towards the photodiodes of said sensor;
- a centring system comprising a four-partitioned diaphragm for housing a finger of a patient; a lever arranged to open the four-partitioned diaphragm; and a spring arranged to close the four-partitioned diaphragm;
- an optical barrier arranged to control the starting of the two diode lasers by passing the finger of the patient;
- a switch-on button for activating the device; and
- a display,
wherein, in use, the working of said device includes the following steps:
- the patient puts a finger into an opening of the device, while operating the lever that opens the four-partitioned diaphragm;
- upon releasing the lever, the spring closes the diaphragm, thus determining a centred arrangement of the finger, irrespective of its dimensions, within the focus of the two laser rays emitted by the two diode lasers and directed towards the photodiodes of said sensor;
- the patient activates the device by pressing the switch-on button, whereby the passing of the finger sets off the optical barrier that controls the starting of the two diode lasers;
- the photodiodes of the sensor read two values of the laser rays resulting from the selective absorption of the laser rays, emitted by the two diode lasers, by the glycate haemoglobin and the free plasmic glucose present in the blood of the patient; and wherein the device further comprises:
- a processor;
- an analogic stage arranged to forward said two values to the processor which is arranged to, after stabilization of said two values, to digitize, to normalize, and to extract a high number of said two values, to strike an average, and, through a particular algorithm, to obtain an instant value of glycaemia, that then appears on the display, wherein in the selective absorption of the rays, emitted by said two diode lasers, by the glycate haemoglobin and the free plasmic glucose, the glycate haemoglobin works as a control parameter, so as to obtain absolutely accurate glycaemia values, and
wherein said two values of the laser radiation resulting from the selective absorption of glycate haemoglobin and free plasmic glucose are calculated in connection with each other, whereby the measurement of the glycaemia is absolutely accurate notwithstanding the thickness and the colour of the skin and the thickness of the patient's finger.

2. The device according to claim 1, wherein the device comprises a memory and two slide keys, and wherein the device is arranged such that, in use, it only needs a few seconds to determine the instantaneous value of glycaemia in an absolutely painless and non-invading method, whereby it can be used to get several glycaemia measurements, even in a very short time range, to monitor the trend through time, to check the effectiveness of a therapy, to build glycaemic curves of newborns, children, adults and elderly people,
wherein the values can be stored in sequence up to 5000 assessments and the memory can be displayed through the two slide keys, and wherein the device comprises a USB gate, a PC connection cable and is provided with a software to visualize and process data.

3. The device according to claim 1 or 2, wherein the device is arranged such that, in use, it is easily activated by pressing the switch-on button, and wherein it is enough to put the patient's finger into the device opening and start the centring system to obtain automatically, after a few seconds, the glycaemia value, that will be displayed on the display together with date and time of each measurement and the progressive registration number.

## Patentansprüche

1. Vorrichtung zur nicht invasiven Bestimmung des Blutzuckerwertes ohne in die Haut des Fingers eines Patienten zu stechen und ohne eine Blutprobe zu nehmen, umfassend:
- zwei Diodenlaserquellen zum Emittieren jeweiliger Laserstrahlen, wobei die zwei Diodenlaserquellen in einem Frequenzbereich zwischen 500 und 1000 nm mit einer Leistung im Bereich zwischen 0,01 und 100 mW bedienbar sind;
- einen Sensor, umfassend Photodioden zum Empfangen der Laserstrahlen, die von den zwei Diodenlasern emittiert werden und in Richtung der Photodioden des Sensors gerichtet sind;
- ein Zentrierungssystem, umfassend eine vierteilige Trennwand zum Aufnehmen eines Fingers eines Patienten; einen Hebel, der für das Öffnen der vierteiligen Trennwand angeordnet ist; und eine Feder, die für das Schließen der vierteiligen Trennwand angeordnet ist;
- eine optische Barriere, die so angeordnet ist, dass sie den Start der zwei Diodenlaser durch Passieren des Fingers des Patienten steuert;
- einen Einschaltknopf zum Aktivieren der Vorrichtung; und
- eine Anzeige,
wobei, im Gebrauch, die Funktionsweise der Vorrichtung die folgenden Schritte umfasst:
- der Patient steckt einen Finger in eine Öffnung der Vorrichtung, während der Hebel betätigt wird, der die vierteilige Trennwand öffnet;
- beim Loslassen des Hebels schließt die Feder die Trennwand, wodurch eine zentrierte Ausrichtung des Fingers ungeachtet seiner Dimensionen im Fokus der zwei Laserstrahlen, die von den zwei Diodenlasern emittiert werden und in Richtung der Photodioden des Sensors gerichtet sind, bestimmt wird;
- der Patient aktiviert die Vorrichtung durch Drücken des Einschaltknopfes, wodurch das Passieren des Fingers die optische Barriere, die den Start der zwei Diodenlaser steuert, auslöst;
- die Photodioden des Sensors erfassen zwei Werte der Laserstrahlen, die aus der selektiven Absorption der Laserstrahlen, die von den zwei Diodenlasern emittiert werden, durch das Glykohämoglobin und die freie Plasmaglucose, die im Blut des Patienten vorliegen, resultieren; und
wobei die Vorrichtung ferner umfasst:
- einen Prozessor;
- eine Analogstufe, die so angeordnet ist, dass sie die zwei Werte an den Prozessor weitergibt, der so angeordnet ist, dass er, nach der Stabilisierung der zwei Werte, eine hohe Zahl der zwei Werte digitalisiert, normalisiert und extrahiert, wodurch ein Mittelwert gebildet und durch einen bestimmten Algorithmus ein gegenwärtiger Blutzuckerwert erhalten wird, der dann auf der Anzeige erscheint,
wobei bei der selektiven Absorption der Strahlen, die von den zwei Diodenlasern emittiert werden, durch das Glykohämoglobin und die freie Plasmaglucose, das Glykohämoglobin als ein Kontrollparameter fungiert, so dass absolut genaue Blutzuckerwerte erhalten werden, und
wobei die zwei Werte der Laserstrahlung, die aus der selektiven Absorption von Glykohämoglobin und freier Plasmaglucose resultieren, in Zusammenhang miteinander berechnet werden, wodurch die Messung des Blutzuckers absolut genau ist, ungeachtet der Dicke und der Farbe der Haut und der Dicke des Fingers des Patienten.

2. Vorrichtung nach Anspruch 1, wobei die Vorrichtung einen Speicher und zwei Schiebetasten umfasst, und wobei die Vorrichtung so angeordnet ist, dass sie, im Gebrauch, nur wenige Sekunden benötigt, um den momentanen Blutzuckerwert mit einem absolut schmerzfreien und nicht invasiven Verfahren zu bestimmen, wodurch sie für den Erhalt mehrerer Blutzuckermessungen verwendet werden kann, selbst in einem sehr kurzen Zeitbereich, um den Trend über die Zeit zu überwachen, um die Wirksamkeit einer Therapie zu überprüfen, um Blutzuckerkurven von Neugeborenen, Kindern, Erwachsenen und älteren Menschen zu erstellen,
wobei die Werte in einer Folge von bis zu 5000 Bewertungen gespeichert werden können und der Speicher mittels der zwei Schiebetasten angezeigt werden kann, und
wobei die Vorrichtung einen USB-Gate, ein PC-Anschlusskabel umfasst und mit einer Software zum Visualisieren und Verarbeiten von Daten ausgestattet ist.

3. Vorrichtung nach Anspruch 1 oder 2, wobei die Vorrichtung so angeordnet ist, dass sie, im Gebrauch, leicht durch Drücken des Einschaltknopfes aktiviert wird, und wobei es ausreicht, den Finger des Patienten in die Öffnung der Vorrichtung zu stecken und das Zentrierungssystem zu starten, um automatisch nach wenigen Sekunden den Blutzuckerwert zu erhalten, der auf der Anzeige zusammen mit dem Datum und der Zeit jeder Messung und der fortlaufenden Registrierungsnummer angezeigt wird.

## Revendications

1. Dispositif pour la détermination non-invasive d'une valeur de glycémie sans piquer la peau d'un doigt d'un patient et sans prélever un échantillon de sang, comprenant :
- deux sources à diode laser, pour émettre des rayons laser respectifs, lesdites deux sources à diode pouvant fonctionner sur un intervalle de fréquence de 500 à 1000 nm, avec une puissance de 0,01 à 100 mW ;
- un capteur comprenant des photodiodes aptes à recevoir les rayons laser émis par les deux diodes lasers, dirigés vers les photodiodes dudit capteur ;
- un système de centrage comprenant un séparateur à quatre parois pour loger un doigt d'un patient ; un levier apte à ouvrir le séparateur à quatre parois ; et un ressort apte à fermer le séparateur à quatre parois ;
- une barrière optique apte à commander l'activation des deux diodes laser en réponse au passage du doigt du patient ;
- un bouton de mise en marche pour actionner le dispositif ; et
- une unité d'affichage,
dans lequel, lors de l'utilisation, le fonctionnement dudit dispositif comprend les étapes suivantes :
- le patient pose son doigt dans une ouverture du dispositif, tout en actionnant le levier qui ouvre le séparateur à parois ;
- une fois le levier relâché, le ressort ferme le séparateur, ce qui détermine un positionnement centré du doigt, indépendamment de ses dimensions, dans le foyer des deux rayons laser émis par les deux diodes laser, dirigés vers les photodiodes dudit capteur ;
- le patient actionne le dispositif en appuyant sur le bouton de mise en marche, le passage du doigt déclenchant la barrière optique qui commande l'activation des deux diodes laser ;
- les photodiodes du capteur lisent les deux valeurs des rayons laser résultant de l'absorption sélective des rayons laser émis parler de diode laser, déterminée par l'hémoglobine glyquée et le glucose plasmatique libre présent dans le sang du patient ; et
dans lequel le dispositif comprend également
- un processeur ;
- un étage analogique apte à retransmettre lesdites deux valeurs au processeur qui est apte, après la stabilisations desdites deux valeurs, à digitaliser, à normaliser et à extraire un nombre élevé desdites deux valeurs pour calculer une moyenne et, par un algorithme particulier, pour obtenir une valeur instantanée de la glycémie, à afficher sur l'unité d'affichage,
dans lequel l'absorption sélective des rayons émis par lesdits deux diodes laser, déterminée par l'hémoglobine glyquée et le glucose plasmatique libre constitue un paramètre de contrôle pour obtenir des valeurs de glycémie absolument précis, et
dans lequel lesdites deux valeurs du rayonnement laser résultant de l'absorption sélective par l'hémoglobine gliquée et le glucose plasmatique libre sont calculées l'une par rapport à l'autre, la mesure de la glycémie étend absolument précise indépendamment de l'épaisseur et de la couleur de la peau et de l'épaisseur du doigt du patient.

2. Dispositif selon la revendication 1, dans lequel le dispositif comprend une mémoire et deux curseurs, et dans lequel le dispositif est réalisé de façon que, lors de l'utilisation, il ne demande que quelques secondes pour déterminer la valeur instantanée de la glycémie, d'une façon absolument indolore et non-invasive, ce qui lui permet d'être utilisé pour obtenir plusieurs mesures de glycémie, même dans un intervalle de temps très court, pour en contrôler la tendance dans le temps, pour vérifier l'efficacité d'une thérapie, pour créer des courbes de glycémie de nouveaux-nés, enfants, adultes et personnes âgées, dans lequel les valeurs peuvent être mémorisées en séquences de jusqu'à 5000 acquisitions, et la mémoire peut être affichée par les deux curseurs, et dans lequel le dispositif comprend un port USB, un câble de connexion avec un PC, et être pourvu d'un logiciel pour l'affichage et le traitement des données.

3. Dispositif selon la revendication 1 2, dans lequel le dispositif est réalisé de façon que, lors de l'utilisation, il est facilement actionné par la pression du bouton de mise à marche, et dans lequel il est suffisant de poser le doigt du patient dans l'ouverture du dispositif est d'actionner le système de centrage pour obtenir automatiquement, après quelques secondes, la valeur de glycémie qui sera affichée sur l'écran du dispositif, avec la date et l'heure de chaque mesure et le numéro d'enregistrement progressif.
